# FASCICULE DE BREVET EUROPEEN

(11) **EP 2 892 940 B1**
(45) Date de publication et mention de la délivrance du brevet: **14.02.2018**
(21) Numéro de dépôt: 13766595.6
(22) Date de dépôt: 29.08.2013
(51) Int. Cl.: A61K 9/00, A61L 27/54, C08K 9/00, A61L 27/36, C08G 63/90, C08J 7/02

(54) **PROCÉDÉ DE TRAITEMENT POUR L'ÉLABORATION D'IMPLANTS OU DE PROTHÈSES DE POLYMÈRES À LIBÉRATION CONTRÔLÉE DE PRINCIPES ACTIFS**
VERFAHREN ZUR HERSTELLUNG VON IMPLANTATEN ODER PROTHESEN AUS POLYMEREN ZUR KONTROLLIERTEN FREISETZUNG VON WIRKSTOFFEN
TREATMENT PROCESS FOR PRODUCING IMPLANTS OR PROSTHESES OF POLYMERS FOR CONTROLLED RELEASE OF ACTIVE INGREDIENTS

(30) Priorité: 04.09.2012 FR 1258210
(43) Date de publication de la demande: 15.07.2015
(73) Titulaire: André, Jean-Marie, 13001 Marseille (FR); Badens, Elisabeth, 13008 Marseille (FR); Forzano, Olivier, 83270 Les Lecques (FR); Masmoudi, Yasmine, 13090 Aix En Provence (FR)
(72) Inventeur: André, Jean-Marie, 13001 Marseille (FR); Badens, Elisabeth, 13008 Marseille (FR); Forzano, Olivier, 83270 Les Lecques (FR); Masmoudi, Yasmine, 13090 Aix En Provence (FR)
(74) Mandataire: Weber, Etienne Nicolas
(86) Numéro de dépôt international: PCT/FR2013/051990
(87) Numéro de publication internationale: WO 2014/037651

(56) Documents cités:
- EP-B1- 0 674 679
- US-A- 5 607 518
- US-A1- 2005 163 852
- US-A1- 2006 008 506
- FERNANDO YAEZ ET AL: "Supercritical fluid-assisted preparation of imprinted contact lenses for drug delivery", ACTA BIOMATERIALIA, ELSEVIER, AMSTERDAM, NL, vol. 7, no. 3, 4 octobre 2010 (2010-10-04) , pages 1019-1030, XP028131193, ISSN: 1742-7061, DOI: 10.1016/J.ACTBIO.2010.10.003 [extrait le 2010-10-08]

## Description

La présente invention concerne un procédé de traitement pour l'élaboration d'implants ou de prothèses de polymères, tel que par exemple des lentilles de contact cornéennes, des implants intraoculaires ou des implants d'ingénierie tissulaire, à libération contrôlée de principes actifs.

Le procédé selon l'invention comprend une première étape dite « de prétraitement » desdits implants ou prothèses, et comprend une seconde étape dite « d'imprégnation » de ces derniers par des principes actifs.

L'étape dite « de prétraitement » est avantageusement mise en oeuvre pour le nettoyage et la stérilisation des implants ou prothèses initialement conditionnés ou non dans une solution physiologique, mais elle peut également être associée ou couplée à une seconde étape visant à imprégner lesdits implants ou prothèses d'actifs pharmaceutiques. Dans le cas second, l'étape de prétraitement devient essentielle au procédé de traitement selon l'invention qui vise l'élaboration d'implants ou de prothèses de polymères capables de libérer une quantité contrôlée de principes actifs. En effet, l'étape de prétraitement permet notamment de disposer d'un matériel avec un état initial reproductible autorisant un contrôle optimal de l'étape d'imprégnation par des principes actifs desdits implants ou prothèses.

Tout implant ou prothèse de polymères destiné à être imprégné de substances actives, est visé, mais on s'intéressera plus particulièrement aux implants intraoculaires (du type de ceux utilisés en chirurgie de la cataracte) et aux lentilles de contact cornéennes souples ou rigides. Entre autres applications, l'invention sera très avantageusement mise en oeuvre pour l'imprégnation des implants intraoculaires et lentilles de contact hydrophiles conservés dans du liquide physiologique ou toute autre solution aqueuse appropriée, ainsi que pour l'imprégnation des implants intraoculaires hydrophobes, sans toutefois que cette mise en oeuvre ne soit exclusivement limitée à ce type d'implants et lentilles.

De manière schématique, l'invention est réalisée par la mise en contact dans une cellule, par exemple un autoclave, des implants ou prothèses polymériques avec un fluide sous pression de type CO₂ en phase supercritique ou subcritique pour éliminer l'eau, les solvants ou autres composés contenus dans lesdits polymères, et indésirables. Dans cette étape, les phases de pressurisation, de mise en contact prolongée des polymères avec le fluide supercritique, et de dépressurisation sont, de façon essentielle à l'invention, entièrement contrôlées et définies. Les polymères ainsi prétraités sont ensuite imprégnés de principes actifs dans une cellule alimentée en CO₂, en mode batch ou en mode continu. Les mêmes précautions de contrôle de la montée et de la descente en pression et/ou température doivent être prises.

Les principes actifs sont des actifs pharmaceutiques, de préférence choisis dans le groupe comprenant les antibiotiques, les anti-inflammatoires stéroïdiens et non-stéroïdiens, les anti-histaminiques, les anti-glaucomateux, les anti-herpétiques, les anti-viraux, les antiseptiques, les anticorps, les anti-VEGF (« Vascular Endothelial Growth Factor ») présentant une activité thérapeutique.

De manière connue, les systèmes implantaires à libération de principes actifs sont des implants capables de libérer, une fois en place sur le patient, des principes actifs (anti-inflammatoires, anti-infectieux, etc.) directement dans la zone « cible », de manière régulière et constante et à une dose prédéterminée, pour traiter ladite zone.

Ces systèmes permettent un traitement du patient plus aisé et contrôlé (le patient n'a plus besoin de respecter un protocole de soins parfois contraignant).

Toutefois, l'élaboration de ces systèmes rencontre plusieurs difficultés :
- La technique d'imprégnation d'implants ou de prothèses polymériques par des principes actifs en phase subcritique ou supercritique est connue, mais présente l'inconvénient majeur d'engendrer un phénomène de moussage ou « foaming », c'est-à-dire la formation de bulles ou pores due au gonflement du polymère sous l'action du fluide sous pression (CO₂ par exemple) seul ou combiné à l'eau présente dans l'implant, puis à sa désorption au cours de la phase de dépressurisation. Plus spécifiquement, les fluides supercritiques et en particulier le CO2 supercritique ont un effet plastifiant sur les matériaux polymériques provoquant leur gonflement. Selon les conditions de mise en contact du fluide supercritique et du matériau polymérique, les propriétés optiques et mécaniques des implants sont altérées, entraînant notamment l'opacification des implants intraoculaires ou la modification de la porosité des implants d'ingénierie tissulaire (« scaffold »). Ce phénomène est amplifié pour les polymères hydratés..
   On connaît par exemple le brevet US 2006/0008506 qui décrit une méthode pour imprégner des articles thérapeutiques ophtalmiques (par exemple des lentilles) par des substances médicamenteuses, en utilisant des fluides comprimés. La mise en oeuvre de ce procédé par les auteurs de la présente invention a démontré que l'eau restée et contenue dans les articles ophtalmiques visés par ledit brevet a tendance à s'extraire desdits articles après un temps de latence indéfini et variable, mais il ne peut jamais être assuré que toute l'eau se soit extraite, à chaque mise en oeuvre du procédé et pour chaque article ophtalmique. Il n'y a donc ainsi ni contrôle de la quantité d'eau extraite - ou a contrario de l'eau restée au sein de l'implant -, ni contrôle de la dose de principes imprégnés. A chaque mise en oeuvre du procédé, les résultats diffèrent. Il n'y a aucune reproductibilité du modèle expérimental.
   S'agissant particulièrement des implants ou prothèses polymériques hydrophiles conservés et conditionnés avant imprégnation dans une solution aqueuse (par exemple du liquide physiologique), le phénomène de moussage est amplifié par la présence de ladite solution au sein de la matrice polymérique. En effet, une certaine quantité de la solution de conditionnement peut pénétrer dans ledit polymère et l'on constate alors que la bonne réalisation de l'étape d'imprégnation est systématiquement entravée par l'eau ayant diffusé au sein de la matrice polymérique. Selon la quantité d'eau retenue par le polymère, la qualité de l'imprégnation et la quantité de principes actifs imprégnés varient d'un implant à un autre, ce qui ne permet pas un réel contrôle de la dose de principes actifs contenue par chaque implant et qui sera ensuite relarguée dans le patient. En outre, lorsqu'il s'agit d'implants ou de prothèses oculaires, la transparence doit être totale pour ne pas gêner la vision, et l'opacité due au phénomène de moussage est totalement exclue.
   Il n'existe pas aujourd'hui de techniques permettant d'obtenir un état initial, stable et reproductible du polymère, avant son imprégnation, et sans dégradation de ses qualités
- Il existe aussi des traitements d'imprégnation des supports polymériques par voie liquide basse pression. Dans ces cas, il y a utilisation comme vecteur d'imprégnation de solvants souvent organiques, qui peuvent rester à l'état de traces résiduelles dans le polymère. Or, ces solvants ont des effets toxiques sur le patient et doivent être évacués en fin de procédé, Il faut donc ajouter au procédé des étapes de traitement supplémentaires, d'une part consommatrices d'énergie et d'autre part incluant souvent une étape de séchage dégradant les substances thermolabiles imprégnées ou constitutives de l'implant ou de la prothèse. Par ailleurs, des traces de solvant résiduel peuvent persister dans l'échantillon et provoquer des réactions inflammatoires sur le patient, comme par exemple, le « syndrome toxique du segment antérieur » dans le cas des implants intraoculaires.

Le procédé selon l'invention a pour but de remédier à ces insuffisances et d'éviter les inconvénients précités. En outre, il permet avantageusement de disposer dans un même temps, d'un matériel implantaire stérile.

Enfin, le procédé selon l'invention permet d'éviter le phénomène de « glistening » (éblouissement) fréquemment observé après la pose sur le patient d'implants intraoculaires de type acryliques hydrophobes. Ces implants polymériques contiennent après leur fabrication de l'eau résiduelle qui a tendance à se vacuoliser et à troubler de manière sérieuse la vision du patient. Le traitement de ces implants par le procédé selon la présente invention et en particulier par l'étape dite « de pré-traitement » permet d'extraire l'eau résiduelle dans sa totalité ce qui supprime alors le phénomène de vacuolisation provoquant le glistening.

Ainsi, le procédé selon l'invention :
- Nettoie efficacement les implants et prothèses polymériques, en extrayant totalement l'eau, les solvants mais également les monomères ou oligomères résiduels contenus dans la matrice polymère ;
- Stérilise lesdits implants et prothèses simultanément et sans ajouter d'étape supplémentaire ;
- Evite le moussage des implants et prothèses lors de leur traitement en milieu supercritique;
- Evite le phénomène de « glistening » apparaissant après implantation chirurgicale des implants intraoculaires acryliques hydrophobes
- Evite la toxicité des implants ou prothèses due au relargage de solvants résiduels, et notamment le « syndrome toxique du segment antérieur » dans le cadre des implants intraoculaires ;
- Assure un état initial reproductible permettant ainsi d'aboutir de manière fiable à des taux d'imprégnation contrôlés de principes actifs après imprégnation des implants et prothèses ;
- Permet une standardisation du matériel implantaire avant imprégnation ;
- Est un procédé écologique ou « vert », facile à mettre en oeuvre, et d'un coût relativement modéré.

Le procédé selon l'invention consiste d'abord à mettre en contact pendant un temps déterminé, un implant ou prothèse polymérique et un fluide sous pression en phase supercritique ou subcritique liquide, de type CO₂ supercritique ou subcritique, dans une cellule permettant la maîtrise des conditions de température et de pression. La montée en pression et dans une moindre mesure la montée en température, doivent être lentes. La dépressurisation, menée de préférence dans des conditions isothermes, doit être opérée avec une cinétique contrôlée et lente. C'est l'étape dite « de prétraitement ».

Pour la bonne mise en oeuvre de l'invention, il est impératif de contrôler les conditions de pressurisation et de dépressurisation, notamment les cinétiques de montée et de descente en pression. Dans une moindre mesure, la température et les conditions de chauffage et de refroidissement peuvent avoir un effet similaire. En effet, si la montée en pression est trop rapide, il y aura altération des propriétés du polymère, même si la cinétique de dépressurisation est lente. De la même manière, si la montée en pression est lente, mais que la cinétique de dépressurisation est rapide, les propriétés seront également altérées.

Le document US 2006/0008506 précité décrit par exemple des conditions de pressurisation à 90 bar et de température à 40°C pendant une période de 30 minutes, suivie d'une dépressurisation lente de la cellule (pendant 1,5 minute). Les conditions de montée en pression n'y sont pas décrites et n'ont pas été étudiées. Or, les auteurs de la présente invention ont démontré que sans contrôle à la fois des conditions de pressurisation et de dépressurisation, le phénomène de moussage est inévitable en raison de la sorption sous l'action du fluide sous pression, du mélange CO2/solutés dans le polymère et/ou de la présence d'eau dans les implants hydratés.

En outre, ce document (ni aucun autre relevé dans l'art antérieur) ne décrit ni ne suggère un traitement des implants ou prothèses de polymère indépendamment et en dehors de toute phase d'imprégnation. Ainsi, le document US 2006/0008506 décrit un traitement des implants par CO2 supercritique en présence d'un médicament (Flubiprofen). Or la présence d'un médicament dont le Flubiprofen joue inévitablement un rôle sur la nature chimique de la phase fluide présente dans la cellule en agissant sur la polarité, les propriétés de diffusivité matière, les tensions interfaciales, etc. des phases en présence, ainsi que sur la thermodynamique du système (le nombre de phases et la nature des phases en présence sont modifiées). La présence du médicament modifie ainsi les conditions d'élimination de l'eau et des solvants résiduels des implants ou prothèses polymériques. D'un implant à un autre, une quantité variable d'eau ou de solvants résiduels demeure, augmentant la dégradation de leur qualité et de leurs propriétés.

Sans l'étape de prétraitement selon l'invention, il ne peut exister de contrôle de la quantité d'eau extraite - ou a contrario de l'eau restée au sein de l'implant -, entraînant ainsi une absence de contrôle de la dose de principes imprégnés.

Pour la réalisation du procédé selon l'invention, la cellule utilisée (de type autoclave), correspond à toute cellule ayant des capacités à résister à des conditions de pression élevée.

Selon une disposition avantageuse de l'invention, l'étape de prétraitement par CO₂ se fait en phase supercritique. Un fluide est dit supercritique lorsqu'il est placé dans des conditions de température et de pression au-delà de son point critique. Les propriétés des fluides supercritiques sont intermédiaires entre celles des liquides et des gaz. Les fluides supercritiques ont une viscosité proche de celle des gaz, une densité proche de celle des liquides, une diffusivité plus élevée que celle des liquides et une tension interfaciale qui tend vers zéro lorsque la pression augmente.

Selon une autre disposition caractéristique de l'invention, l'étape de prétraitement par CO₂ peut se faire dans des conditions subcritiques. Un fluide est dit subcritique lorsque qu'il est soumis à une température inférieure à la température critique dudit fluide.

Lorsque l'étape de prétraitement s'achève, on dispose d'un matériel exempt de traces résiduelles de solvant, d'eau, éventuellement de monomères, et stérile. Il est alors possible de poursuivre le procédé de traitement par la phase d'imprégnation des implants ou prothèses par des actifs pharmaceutiques.

La mise en oeuvre du procédé selon l'invention est effectuée en mode batch ou en mode continu.

Le mode batch, ou discontinu, est un système de production en fonctionnement discontinu, calqué sur le principe du traitement par lots. Dans le cas de l'imprégnation, ce régime de fonctionnement permet une économie de principe actifs et de CO₂ utilisés. En mode continu, le fluide (CO₂) traverse en continu l'autoclave (il est introduit dans la cellule et est évacué en continu).

Les buts, avantages et caractéristiques du procédé selon l'invention ressortiront mieux de la description qui suit et des dessins annexés dans lesquels :
- La figure 1 est une vue schématique d'un exemple de banc expérimental utilisé pour la mise en oeuvre de l'étape de prétraitement selon l'invention.
- La figure 2 est une vue schématique d'un exemple de banc expérimental utilisé pour l'étape d'imprégnation en continu des implants polymériques selon l'invention.

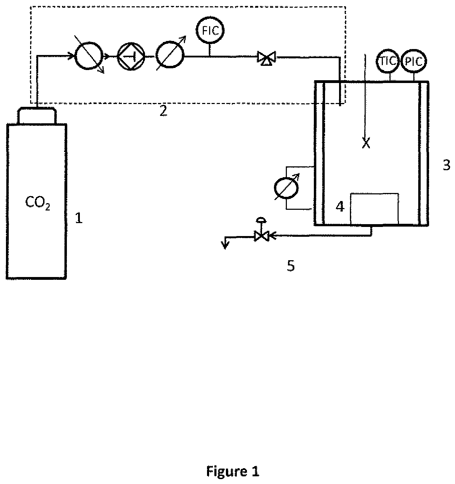

Selon un premier mode préféré d'exécution de l'invention (figure 1), l'étape de prétraitement d'implants ou de prothèses de polymères comprend, en mode batch, les phases de réalisation suivantes :
- Les matrices polymériques, c'est-à-dire les implants ou prothèses de polymères à traiter en vue de leur imprégnation, sont introduites dans une cellule haute pression ou autoclave (3) dans laquelle elles sont de préférence déposées sur un support adapté à les recevoir (4).
- La cellule est ensuite alimentée de manière contrôlée (faible taux de pressurisation) en CO₂. Le CO₂ est issu d'une réserve, par exemple une cuve ou une bouteille (1), et conduit à la cellule par une ligne d'alimentation (2).
- La cellule est chauffée avec une vitesse de chauffe contrôlée jusqu'à atteindre les conditions subcritiques liquides ou supercritiques du CO₂, avec une pression comprise entre 5 et 20MPa et une température entre 20°C et 50°C. Le taux de pressurisation est choisi en fonction de la nature de la matrice polymérique et est de préférence compris entre 0,01 et 1MPa/min pour la cellule d'imprégnation.
- La cellule haute pression ou autoclave est isolée pendant une durée de quelques minutes à une heure. Le CO₂ en contact avec les matrices polymériques solubilise l'eau, les solvants, les monomères ou oligomères résiduels (ainsi que toute autre substance indésirable que lesdites matrices peuvent contenir).
- La cellule haute pression ou autoclave est ensuite dépressurisée lentement dans des conditions isothermes par l'intermédiaire de la ligne de dépressurisation (5). L'étape de dépressurisation doit être menée lentement pour éviter le phénomène de moussage des polymères. Le taux de dépressurisation est choisi en fonction de la nature de la matrice polymérique et est de préférence compris entre 0,01 et 1MPa/min.
- Les cycles de pressurisation/dépressurisation de la cellule haute pression peuvent être répétés, de 1 à 5 fois.

Cet exemple de réalisation permet d'extraire l'eau des implants dans sa totalité, ainsi que les solvants, les monomères résiduels et toute autre substance indésirable, et optimise au mieux le contrôle de la phase d'imprégnation aussi en bien en termes d'efficacité d'imprégnation que du maintien des propriétés optiques et mécaniques des implants. Ce mode de réalisation consomme également peu de CO₂.

Selon un deuxième mode de réalisation, l'étape de prétraitement des implants ou des prothèses de polymères se déroule en mode continu. La mise en oeuvre du procédé comprend alors les phases suivantes :
- Les matrices polymériques, c'est-à-dire implants ou prothèse de polymères, à traiter en vue de leur imprégnation, sont introduites dans la cellule haute pression ou autoclave, et sont, de préférence, déposées sur un support adéquat.
- La cellule est ensuite alimentée en CO₂ de manière contrôlée (faible taux de pressurisation) et chauffée avec une vitesse de chauffe contrôlée jusqu'à atteindre les conditions supercritiques ou subcritiques liquides du CO₂, avec une pression comprise entre 5 et 20MPa et une température entre 20°C et 50°C. Le taux de pressurisation est choisi en fonction de la nature de la matrice polymérique et est de préférence compris entre 0,01 et 1MPa/min pour la cellule d'imprégnation.
- Un flux de CO₂ traverse ensuite la cellule haute pression pendant une durée pouvant varier de quelques minutes à 1 heure. Le débit de CO₂ choisi est compris dans une gamme de débits allant de quelques grammes par heure à 1kg/h.
- La cellule haute pression ou autoclave est ensuite dépressurisée lentement dans des conditions isothermes. Le taux de dépressurisation varie en fonction de la nature de la matrice polymérique et est de préférence compris entre 0,01 et 1MPa/min.

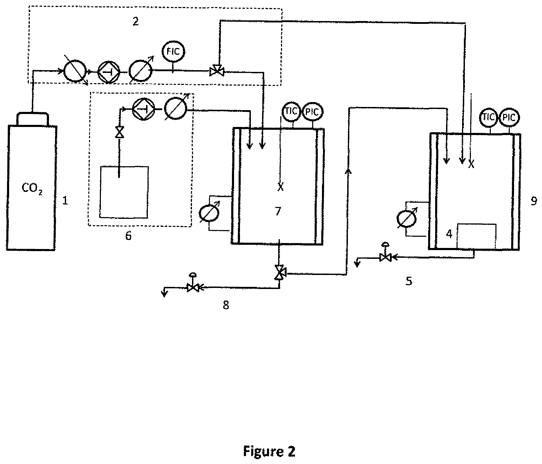

L'étape de prétraitement par CO₂ des polymères permet d'obtenir un matériel stérile exempt de toutes substances susceptibles de gêner la complète réalisation de la phase d'imprégnation par principes actifs. Le relargage de composés toxiques chez le patient est évité. Cette étape peut être suivie de l'étape d'imprégnation en phase supercritique qui peut être également menée en mode batch ou en mode continu. Les conditions de montée ou descente en pression doivent également être contrôlées durant cette phase d'imprégnation pour éviter le moussage. Les implants hydrophiles étant sensibles au phénomène de moussage, l'extraction de l'eau résiduelle lors de l'étape de pré-traitement préalable permet également de limiter le phénomène de moussage au cours de l'imprégnation,
- En mode batch, l'étape d'imprégnation comprend les phases successives suivantes :
   a) les implants ou prothèses de polymères prétraités sont introduits dans une cellule haute pression de type autoclave et sont de préférence déposés sur tout support adapté à leur réception.
   b) Quelques milligrammes à quelques grammes de principes actifs sont déposés dans ladite cellule ou autoclave. Les principes actifs sont des actifs pharmaceutiques, de préférence choisis dans le groupe comprenant les antibiotiques, les anti-inflammatoires stéroïdiens et non-stéroïdiens, les anti-histaminiques, les anti-glaucomateux, les anti-herpétiques, les anti-viraux, les antiseptiques, les anticorps, les anti-VEGF (« Vascular Endothelial Growth Factor ») présentant une activité thérapeutique.
   c) Selon la nature du principe actif à imprégner, 1 à 15 mol.% par rapport au CO₂ d'un cosolvant de type connu (par exemple : éthanol) sont introduits dans l'autoclave par une ligne d'alimentation dédiée, et un piège à solvant est installé dans la ligne d'évacuation.
   d) La cellule est ensuite alimentée en CO₂ de manière contrôlée (faible taux de pressurisation) et chauffée avec une vitesse de chauffe contrôlée jusqu'à atteindre les conditions supercritiques ou subcritiques liquides du CO₂, avec une pression comprise entre 5 et 20MPa et la température entre 20°C et 50°C. Le taux de pressurisation est choisi en fonction de la nature de la matrice polymérique et est de préférence compris entre 0,01 et 1MPa/min pour la cellule d'imprégnation.
   e) La cellule haute pression ou autoclave est isolée pendant une durée de 30 minutes à 5 heures. Les principes actifs se solubilisent dans la phase supercritique riche en CO₂ et le mélange diffuse à l'intérieur des matrices polymériques.
   f) Si un cosolvant est utilisé, une étape de lavage, préalable à la dépressurisation, est effectuée par le passage dans la cellule d'imprégnation d'un flux de CO₂ à débit constant, de quelques grammes par heure à 1kg/h, pendant une à 5 fois le temps de passage.
   g) La cellule haute pression ou autoclave est ensuite dépressurisée lentement dans des conditions isothermes. Le taux de dépressurisation est choisi en fonction de la nature de la matrice polymérique et est de préférence compris entre 0,01 et 1MPa/min.
- En mode continu (figure 2) :
   En mode continu, le banc expérimental doit être modifié : une seconde cellule haute pression jouant le rôle de saturateur (7) doit être ajoutée en amont de la cellule d'imprégnation. Un saturateur est un appareil utilisé pour solubiliser, généralement à saturation, des solutés dans une phase fluide. Si un cosolvant est utilisé (par exemple de l'éthanol), le saturateur doit être équipé d'une ligne d'alimentation en cosolvant (6). En mode continu, l'étape d'imprégnation comprend les phases successives suivantes :
      A. Les matrices polymériques prétraitées sont introduites dans une cellule haute pression de type autoclave dite « cellule d'imprégnation » (9) et sont de préférence déposées sur tout support adapté à leur réception (4).
      B. Quelques milligrammes à quelques grammes de principes actifs sont déposés dans le saturateur (7).
      C. La cellule d'imprégnation et le saturateur sont ensuite alimentés en CO₂ de manière contrôlée (faible taux de pressurisation) et chauffés avec une vitesse de chauffe contrôlée jusqu'à atteindre les conditions supercritiques ou subcritiques liquides du CO₂, avec une pression comprise entre 5 et 20MPa et une température entre 20°C et 50°C. Le taux de pressurisation est choisi en fonction de la nature de la matrice polymérique et est de préférence compris entre 0,01 et 1MPa/min pour la cellule d'imprégnation. Le CO₂ est issu d'une réserve, par exemple une cuve ou une bouteille (1), et conduit auxdites cellules par une ligne d'alimentation (2).
      D. Un flux de CO₂ constant traverse le saturateur où il solubilise les principes actifs. Le CO₂ chargé en principes actifs (flux CO₂/ principes actifs) à la sortie du saturateur balaie alors la cellule d'imprégnation où il est en contact avec les matrices polymériques. Le débit de CO₂ peut varier entre quelques grammes par heure et 1kg/h. Cette étape a une durée variant de 30 minutes à 5 heures.
      E. Si un cosolvant est utilisé, un débit de cosolvant constant doit alimenter le saturateur pendant 30 minutes à 5 heures, dans un rapport molaire cosolvant/CO₂ compris entre 1 et 15 mol.%. A la sortie du saturateur, le flux CO₂/cosolvant/principes actifs traverse alors la cellule d'imprégnation où il est en contact avec les matrices polymériques.
      F. Si un cosolvant est utilisé, une étape de lavage, préalable à la dépressurisation, est effectuée par le passage dans la cellule d'imprégnation d'un flux de CO₂ à débit constant, de quelques grammes par heure à 1kg/h, pendant une à 5 fois le temps de passage.
      G. La cellule d'imprégnation et le saturateur sont ensuite dépressurisés lentement dans des conditions isothermes par l'intermédiaire respectivement des lignes de dépressurisation 5 et 8. Le taux de dépressurisation est choisi en fonction de la nature de la matrice polymérique et est de préférence compris entre 0,01 et 1MPa/min pour la cellule d'imprégnation.

Dans le procédé précédemment décrit, le cosolvant utilisé à la phase E peut de manière alternative être introduit dans la cellule haute pression avant la montée en pression de ladite cellule, soit au début de la phase C.

Enfin, selon un autre mode de réalisation, l'étape de prétraitement par fluide sous pression peut être couplée à l'étape d'imprégnation en mode batch ou en mode continu avec des conditions opératoires (pression, température, débits, durée) qui peuvent être les mêmes ou différentes pour les deux étapes. Le couplage des 2 étapes nécessite l'utilisation d'un saturateur et le montage expérimental est similaire à celui illustré en figure 2. Les matrices polymériques sont alors chargées dans la cellule d'imprégnation alors que les principes actifs sont chargés dans le saturateur.

L'étape de prétraitement est conduite dans la cellule d'imprégnation comme décrit précédemment, en mode batch ou en mode continu, en contournant le saturateur et en évitant l'étape de dépressurisation. A la fin de la durée de prétraitement, l'étape d'imprégnation peut commencer et être conduite aussi bien en mode batch qu'en mode continu.

En mode batch, le saturateur chargé au préalable en principes actifs et isolé de la cellule d'imprégnation est alimenté en CO₂ jusqu'à atteindre les conditions de pression et de températures désirées. Cette étape peut être menée en parallèle avec l'étape de prétraitement.

Le saturateur et la cellule d'imprégnation sont ensuite connectés pendant une durée prédéterminée de 30 minutes à 5 heures. Dans le cas de l'utilisation de cosolvant, une étape de lavage préalable à la dépressurisation est nécessaire, comme décrit précédemment.

Le procédé de traitement en milieu supercritique pour l'élaboration d'implants ou de prothèses de polymères à libération contrôlée de principes actifs est innovant en ce qu'il comprend une première phase de pressurisation avec une cinétique contrôlée, couplée avec une phase de traitement en milieu supercritique ou subcritique et une phase de dépressurisation suivant également une cinétique contrôlée. Ce prétraitement innovant, réalisé avant toute phase d'imprégnation donc en dehors de toute présence de médicaments, permet de nettoyer les implants ou les prothèses de polymères, en en extrayant les substances indésirables (eau, solvants et monomères résiduels, ...) et en les stérilisant. Le phénomène de vacuolisation fréquemment observé pour certaines catégories d'implants peut être évité. Ce prétraitement assure un état initial des implants ou prothèses reproductible, permettant ainsi d'aboutir à des taux d'imprégnation en principes actifs contrôlés.

## Revendications

1. Procédé de traitement pour l'élaboration d'implants ou de prothèses de polymères à libération contrôlée de principes actifs comprenant une première étape dite « de prétraitement », suivie d'une étape dite "d'imprégnation" des implants ou prothèses de polymères prétraités de principes actifs,
la première étape comportant :
- l'introduction des Implants ou prothèses de polymères dans une cellule haute pression ou autoclave,
- l'alimentation de la cellule en CO₂ dans une phase de pressurisation et de chauffage suivant une cinétique contrôlée avec un taux de pressurisation compris entre 0,01 et 1MPa/min, jusqu'à atteindre des conditions supercritiques ou subcritiques liquides du CO₂, avec une pression comprise entre 5 et 20MPa et une température entre 20°C et 50°C,
- une phase de mise en contact des implants ou prothèses de polymères avec le CO₂ en mode batch ou en mode continu, la phase de mise en contact comprenant :
• dans le mode batch : l'Isolation de la cellule haute pression ou autoclave pendant une durée de quelques minutes à une heure, et
• dans le mode continu : le balayage de la cellule haute pression ou autoclave par un flux traversant de CO₂ pendant une durée variant de quelques minutes à 1 heure, le débit de CO₂ étant compris dans une gamme de débits allant de quelques grammes par heures à 1 Kg/h,
et
- une phase de dépressurisation suivant une cinétique contrôlée avec un taux de dépressurisation compris entre 0,01 et 1 MPa/min, dans des conditions isothermes.

2. Procédé de traitement selon la revendication 1, **caractérisé en ce que**, lors de la première étape de prétraitement, les cycles de pressurisation-dépressurisation de la cellule haute pression sont répétés de 1 à 5 fois.

3. Procédé de traitement selon l'une quelconque des revendications 1 ou 2 **caractérisé en ce que** la seconde étape dite « d'imprégnation », conduite en mode batch et comprend les phases successives suivantes :
a) Les implants ou prothèses de polymères prétraités sont introduits dans une cellule haute pression de type autoclave
b) Quelques milligrammes à quelques grammes de principes actifs sont déposés dans ladite cellule ou autoclave
d) La cellule est ensuite alimentée en CO₂ de manière contrôlée suivant un faible taux de pressurisation et chauffée avec une vitesse de chauffe contrôlée jusqu'à atteindre les conditions supercritiques ou subcritiques liquides du CO₂, avec une pression comprise entre 5 et 20MPa et une température entre 20 et 50°C
e) La cellule haute pression ou autoclave est isolée pendant une durée de 30 minutes à 5 heures
g) La cellule haute pression ou autoclave est ensuite dépressurisée lentement, dans des conditions isothermes.

4. Procédé de traitement selon la revendication 3, **caractérisé en ce que** :
- la phase d) est précédée d'une phase c) dans laquelle 1 à 15 mol.% par rapport au CO₂ d'un cosolvant sont introduits dans l'autoclave, et
- la phase g) est précédée d'une phase f) dans laquelle : une étape de lavage, est effectuée par le passage dans la cellule d'imprégnation d'un flux de CO₂ à débit constant, de quelques grammes par heure à 1kg/h, pendant une à 5 fois le temps de passage

5. Procédé de traitement selon l'une quelconque des revendications 1 à 2, **caractérisé en ce que** la seconde étape dite « d'imprégnation », conduite en mode continu et comprend les phases suivantes :
A. Les implants ou prothèses de polymères prétraités sont introduits dans une cellule haute pression de type autoclave dite « cellule d'imprégnation »
B. Quelques milligrammes à quelques grammes de principes actifs sont déposés dans un saturateur
C. La cellule d'imprégnation et le saturateur sont ensuite alimentés en CO₂ de manière contrôlée suivant un faible taux de pressurisation et chauffés avec une vitesse de chauffe contrôlée jusqu'à atteindre les conditions supercritiques ou subcritiques liquides du CO₂, avec une pression comprise entre 5 et 20MPa et une température entre 20°C et 50°C
D. Le flux CO₂/principes actifs traverse la cellule d'imprégnation où il est en contact avec les matrices polymériques, le débit de CO₂ variant de quelques grammes par heure à 1kg/h, pendant une durée variant de 30 minutes à 5 heures
G. La cellule d'imprégnation et le saturateur sont ensuite dépressurisés lentement dans des conditions isothermes.

6. Procédé de traitement selon la revendication 5, **caractérisé en ce que** la phase D. est suivie :
• d'une phase E. dans laquelle : un débit constant d'un cosolvant alimente le saturateur dans un rapport molaire cosolvant/CO2 de 1 à 15 mol.%, pendant une durée de 30 minutes à 5 heures, et le flux CO₂/cosolvant/principes actifs traverse la cellule d'imprégnation où il est en contact avec les matrices polymériques
• puis d'une phase F. dans laquelle : une étape de lavage est effectuée par le passage dans la cellule d'imprégnation d'un flux de CO₂ à débit constant, de quelques grammes par heure à 1kg/h, pendant une à 5 fois le temps de passage

7. Procédé de traitement selon les revendications 4 ou 6 **caractérisé en ce que** le cosolvant est de l'éthanol.

8. Procédé de traitement selon l'une quelconque des revendications précédentes **caractérisé en ce que** les principes actifs sont choisis dans le groupe comprenant les antibiotiques, les anti-inflammatoires stéroïdiens et non-stéroïdiens, les anti-histaminiques, les anti-glaucomateux, les anti-herpétiques, les anti-viraux, les antiseptiques, les anticorps, les anti-VEGF (« VascularEndothelialGrowth Factor ») présentant une activité thérapeutique.

9. Procédé de traitement pour l'élaboration d'implants ou de prothèses de polymères à libération contrôlée de principes actifs selon l'une quelconque des revendications précédentes, **caractérisé en ce que** les implants ou prothèses de polymères sont des implants intraoculaires ou des lentilles de contact cornéennes souples ou rigides.

## Patentansprüche

1. Verfahren zur Herstellung von Implantaten oder Prothesen aus Polymeren zur kontrollierten Freisetzung von Wirkstoffen, umfassend einen als Vorbehandlung" bezeichneten ersten Schritt, gefolgt von einem als "Imprägnierung" von mit Wirkstoffen vorbehandelten Implantaten oder Prothesen aus Polymeren bezeichneten Schritt, wobei der erste Schritt Folgendes umfasst:
- Einführen von Implantaten oder Prothesen aus Polymeren in eine Hochdruck- oder Autoklavenzelle,
- Versorgen der Zelle mit CO₂ in einer Druckbeaufschlagungs- und Erwärmungsphase gemäß einer kontrollierten Kinetik mit einer Druckbeaufschlagungsrate zwischen 0,01 und 1 MPa/min bis zum Erreichen von superkritischen oder subkritischen Flüssigbedingungen des CO₂ mit einem Druck zwischen 5 und 20 MPa und einer Temperatur zwischen 20 °C und 50 °C,
- eine Phase der Kontaktierung der Implantate oder Prothesen aus Polymeren mit dem CO₂ in einem Batch-Modus oder in einem kontinuierlichen Modus, wobei die Phase der Kontaktierung Folgendes umfasst:
• im Batch-Modus: Isolieren der Hochdruck- oder Autoklavenzelle über eine Dauer von einigen Minuten bis einer Stunde, und
• im kontinuierlichen Modus: Abtasten der Hochdruck- oder Autoklavenzelle durch einen durchlaufenden CO₂-Fluss über eine Dauer, die zwischen einigen Minuten und 1 Stunde variiert, wobei der Durchfluss von CO₂ in einer Spanne von Durchflüssen enthalten ist, die von einigen Gramm pro Stunde bis 1 kg/h reicht, und
- eine Druckabfallphase gemäß einer kontrollierten Kinetik mit einer Druckabfallrate zwischen 0,01 und 1 MPa/min unter isothermen Bedingungen.

2. Verfahren nach Anspruch 1, **dadurch gekennzeichnet, dass** während des ersten Vorbehandlungsschritts die Zyklen von Druckbeaufschlagung/Druckabfall der Hochdruckphase 1- bis 5-mal wiederholt werden.

3. Verfahren nach einem der Ansprüche 1 oder 2, **dadurch gekennzeichnet, dass** der zweite, als "imprägnierung" bezeichnete Schritt im Batch-Modus durchgeführt wird und die folgenden aufeinanderfolgenden Phasen umfasst:
a) die vorbehandelten Implantate oder Prothesen aus Polymeren werden in eine Hochdruckzelle nach Art eines Autoklaven eingeführt,
b) einige Milligramm bis einige Gramm an Wirkstoffen werden in der Zelle oder dem Autoklaven abgelagert,
d) die Zelle wird anschließend gemäß einer schwachen Druckbeaufschlagungsrate auf kontrollierte Weise mit CO₂ versorgt und in einer kontrollierten Erwärmungsgeschwindigkeit erwärmt, bis sie die superkritischen oder subkritischen CO₂-Flüssigbedingungen mit einem Druck zwischen 5 und 20 MPa und einer Temperatur zwischen 20 und 50 °C erreicht,
e) die Hochdruck- oder Autoklavenzelle wird über eine Dauer von 30 Minuten bis 5 Stunden isoliert,
g) die Hochdruck- oder Autoklavenzelle wird anschließend unter isothermen Bedingungen langsam drucklos gemacht.

4. Verfahren nach Anspruch 3, **dadurch gekennzeichnet, dass**:
- der Phase d) eine Phase c) vorausgeht, in der 1 bis 15 Mol-% CO₂ eines Co-Lösungsmittels in den Autoklaven eingeführt werden, und
- der Phase g) eine Phase f) vorausgeht, in der: durch das ein- bis 5-malige Durchleiten eines CO₂-Flusses von einigen Gramm pro Stunde bis 1 kg/h bei konstanter Durchflussmenge ein Waschschritt in der Imprägnierzelle durchgeführt wird.

5. Verfahren nach einem der Ansprüche 1 bis 2, **dadurch gekennzeichnet, dass** der zweite Schritt, der als "Imprägnierung" bezeichnet wird, im kontinuierlichen Modus durchgeführt wird und die folgenden Phasen umfasst:
A. die vorbehandelten Implantate oder Prothesen aus Polymeren werden in eine Hochdruckzelle nach Art eines Autoklaven, als "Imprägnierzelle" bezeichnet, eingeführt,
B. einige Milligramm bis einige Gramm an Wirkstoffen werden in einem Sättiger abgelagert,
C. die Imprägnierzelle und der Sättiger werden anschließend auf kontrollierte Weise gemäß einer schwachen Druckbeaufschlagungsrate mit CO₂ versorgt und in einer kontrollierten Erwärmungsgeschwindigkeit erwärmt, bis die superkritischen oder subkritischen Flüssigbedingungen des CO₂ mit einem Druck zwischen 5 und 20 MPa und einer Temperatur zwischen 20 °C und 50 °C erreicht werden,
D. der Fluss aus CO₂/Wirkstoffen durchläuft die Imprägnierzelle, wo er in Kontakt mit den Polymermatrizen ist, wobei die Durchflussmenge von CO₂ von einigen Gramm pro Stunde bis 1 kg/h variiert, über eine Dauer, die zwischen 30 Minuten und 5 Stunden variiert,
G. die Imprägnierzelle und der Sättiger werden anschließend unter isothermen Bedingungen langsam drucklos gemacht.

6. Verfahren nach Anspruch 5, **dadurch gekennzeichnet, dass** der Phase D Folgendes folgt:
• eine Phase E, in der: eine konstante Durchflussmenge eines Co-Lösungsmittels den Sättiger in einem Molverhältnis von Co-Lösungsmittel/CO2 von 1 bis 15 Mol-% über eine Dauer von 30 Minuten bis 5 Stunden versorgt und der Fluss aus CO₂/Co-Lösungsmittel/Wirkstoffen die Imprägnierzelle durchläuft, wo er mit den Polymermatrizen in Kontakt ist;
• anschließend eine Phase F, in der: durch das ein- bis 5-malige Durchleiten eines CO₂-Flusses von einigen Gramm pro Stunde bis 1 kg/h bei konstanter Durchflussmenge ein Waschschritt in der Imprägnierzelle durchgeführt wird.

7. Verfahren nach Anspruch 4 oder 6, **dadurch gekennzeichnet, dass** das Co-Lösungsmittel Ethanol ist.

8. Verfahren nach einem der vorhergehenden Ansprüche, **dadurch gekennzeichnet, dass** die Wirkstoffe aus der Gruppe ausgewählt sind, die Antibiotika, entzündungshemmende Steroide und Nichtsteroide, Antihistaminika, antiglaukomatöse Wirkstoffe, Antiherpeswirkstoffe, antivirale Wirkstoffe, Antiseptika, Antikörper, Anti-VEGF ("Vascular Endothelial Growth Factor"), die eine therapeutische Wirkung aufweisen, umfasst.

9. Verfahren zur Herstellung von Implantaten oder Prothesen aus Polymeren zur kontrollierten Freisetzung von Wirkstoffen nach einem der vorhergehenden Ansprüche, **dadurch gekennzeichnet, dass** die Implantate oder Prothesen aus Polymeren intraokulare Implantate oder weiche oder harte Kontaktlinsen sind.

## Claims

1. A treatment process for the production of implants or prostheses of polymers with controlled release of active ingredients comprising a first step referred to as a "pretreatment" step followed by a step referred to as a "impregnation" step of the implants or prostheses of polymers pretreated with active ingredients,
the first step comprising:
- introduction of the implants or prostheses of polymers into a high-pressure cell or autoclave,
- feeding the cell with CO₂ in a pressurisation and heating phase in accordance with controlled kinetics with a pressurisation rate of between 0.01 and 1 MPa/min until attaining supercritical or subcritical liquid conditions of the CO₂ with a pressure of between 5 and 20 MPa and a temperature of between 20°C and 50°C,
- a phase of bringing the implants or prostheses of polymers into contact with the CO₂ in a batch mode or a continuous mode, the contacting phase comprising:
• in the batch mode: isolation of the high-pressure cell or autoclave for a period of a few minutes to an hour, and
• in the continuous mode: sweeping the high-pressure cell or autoclave with a through flow of CO₂ for a period varying from a few minutes to one hour, the CO₂ flow rate being in a range of flow rates ranging from a few grams per hour to 1 kg/h, and
- a depressurisation phase in accordance with controlled kinetics with a depressurisation rate of between 0.01 and 1 MPa/min under isothermal conditions.

2. A treatment process according to claim 1 **characterised in that** in the first pretreatment step the high-pressure cell pressurisation-depressurisation cycles are repeated from 1 to 5 times.

3. A treatment process according to either one of claims 1 and 2 **characterised in that** the second "impregnation" step carried out in the batch mode comprises the following successive phases:
a) the pretreated polymer implants or prostheses are introduced into a high-pressure cell of autoclave type,
b) a few milligrams to a few grams of active Ingredients are deposited in said cell or autoclave,
d) the cell is then fed with CO₂ in controlled fashion in accordance with a low pressurisation rate and heated at a controlled heating rate until attaining the supercritical or subcritical liquid conditions of the CO₂ with a pressure of between 5 and 20 MPa and a temperature of between 20 and 50°C,
e) the high-pressure cell or autoclave is isolated for a period of 30 minutes to 5 hours, and
g) the high-pressure cell or autoclave is then slowly depressurised under isothermal conditions.

4. A treatment process according to claim 3 **characterised in that**:
- phase d) is preceded by a phase c) in which 1 to 15 molar % with respect to the CO₂ of a cosolvent are Introduced into the autoclave, and
- phase g) is preceded by a phase f) in which: a washing step is effected by passing into the Impregnation cell a flow of CO₂ at a constant flow rate of a few grams per hour to 1 kg/h for one to 5 times the flow passage time.

5. A treatment process according to either one of claims 1 and 2 **characterised in that** the second "impregnation" step carried out in the continuous mode comprises the following phases:
A. The pretreated polymer implants or prostheses are introduced into a high-pressure cell of autoclave type referred to as the "impregnation cell",
B. A few milligrams to a few grams of active ingredients are deposited in a saturator,
C. The impregnation cell and the saturator are then fed with CO₂ in a controlled manner in accordance with a low pressurisation rate and heated at a controlled heating rate until attaining the supercritical or subcritical liquid conditions of the CO₂ with a pressure of between 5 and 20 MPa and a temperature of between 20°C and 50°C,
D.The CO₂/active Ingredients flow passes through the impregnation cell where it is in contact with the polymeric matrices, the CO₂ flow rate varying from a few grams per hour to 1 kg/h for a period varying from 30 minutes to 5 hours,
G. The impregnation cell and the saturator are then slowly depressurised under isothermal conditions.

6. A treatment process according to claim 5 **characterised in that** phase D is followed by:
• a phase E in which: a constant flow rate of a cosolvent feeds the saturator in a cosolvent/CO₂ molar ratio of 1 to 15 molar % for a period of 30 minutes to 5 hours and the CO₂/cosolvent/active ingredients flow passes through the impregnation cell where it is in contact with the polymeric matrices,
• then a phase F in which: a washing step is carried out by passing Into the impregnation cell a flow of CO₂ at a constant flow rate of a few grams per hour to 1 kg/h for one to 5 times the flow passage time.

7. A treatment process according to claims 4 or 6 **characterised in that** the cosolvent is ethanol.

8. A treatment process according to any one of the preceding claims **characterised in that** the active ingredients are selected from the group comprising antibiotics, steroidal and non-steroidal anti-inflammatories, anti-histamines, anti-glaucoma agents, anti-herpetic agents, anti-viral agents, antiseptics, antibodies, anti-VEGF (vascular endothelial growth factor) having a therapeutic activity.

9. A treatment process for the production of implants or prostheses of polymers with controlled release of active ingredients according to any one of the preceding claims **characterised in that** the polymer implants or prostheses are intraocular implants or flexible or rigid corneal contact lenses.
